# EUROPEAN PATENT SPECIFICATION

(11) **EP 1 317 925 B1**
(45) Date of publication and mention of the grant of the patent: **01.09.2004**
(21) Application number: 02027185.4
(22) Date of filing: 05.12.2002
(51) Int. Cl.: A61K 31/575, A61K 9/16

(54) **Microgranules of Ursodeoxycholic acid**
Mikrogranulate von Ursodeoxycholsäure
Microgranules de l'acide ursodesoxycholique

(30) Priority: 06.12.2001 IT MI20012572
(43) Date of publication of application: 11.06.2003
(73) Proprietor: ISTITUTO BIOCHIMICO ITALIANO GIOVANNI LORENZINI S.p.A., 20134 Milan (IT)
(72) Inventor: Cecchetelli, Loredana, 00045 Genzano (IT); Di Rella, Mario, 00042 Anzio (IT); Tedeschi, Donatella, 20133 Milano (IT)
(74) Representative: Gervasi, Gemma, Dr.

(56) References cited:
- EP-A- 0 509 335
- EP-A- 0 571 338
- EP-A- 0 599 282
- WO-A-00/48577

## Description

### State of the art

Bile acids and in particular ursodeoxycholic acid are efficacious in the treatment of biliary dyspepsia, biliary cirrhosis, and chronic cholestatic hepatopathies.

A major drawback in the therapeutic use of said active ingredients is that they are very bitter and said taste cannot be adequately masked by the addition of sweeteners and/or flavouring agents. It follows that the bile acid formulations found in commerce are predominantly in the form of capsules or tablets to be swallowed, which, however, cannot be used in paediatric treatments or by patients unable to swallow.

A technique already known to improve the palatability of an active ingredient with an unpleasant taste consists in coating the active ingredient formulations with organic polymers. However, in the case of bile acids, the use of said technique is problematic because their bitter taste is particularly pungent and because, due to their poor solubility, a polymeric coating might modify their dissolution profile and jeopardise their bioavailability.

EP-A-0 599 282 discloses microgranules comprising ursodeoxycholic acid, a binder and a plasticizer. It differs from the subject-matter of the present application in that it is explicitly stated that no polymers are added.

Therefore, the need for developing ursodeoxycholic acid oral formulations with a pleasant taste, suitable for administration in paediatric treatments or to patients having swallowing problems, as well as a good bioavailability, is deeply felt.

### Summary

The Applicant has now surprisingly found that microgranules of ursodeoxycholic acid, in which the active ingredient is intimately mixed with a plasticiser and a copolymer of (meth)acrylic acid, are characterised by good palatability and by a bioavailability substantially identical with that of active ingredient formulations already available in commerce. Said microgranules are particularly suitable for the preparation of formulations for oral administration, such as suspensions or chewable tablets.

### Brief description of the Figure

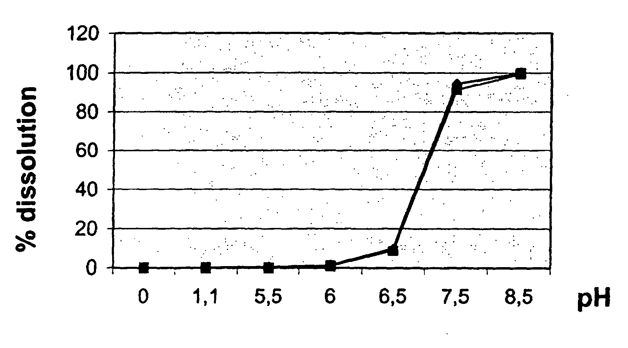

Figure 1 shows the dissolution (%) of ursodeoxycholic acid microgranules and of pure ursodeoxycholic acid at different pH values for the samples prepared as per Example 7.
- ◆- indicates the curve obtained for pure ursodeoxycholic acid
- ■- indicates the curve obtained for ursodeoxycholic acid microgranules

### Detailed description of the invention

It is a subject of the present invention to provide microgranules for the oral administration of ursodeoxycholic acid, characterised in that they comprise, intimately mixed, ursodeoxycholic acid, a plasticiser and a pharmaceutically acceptable copolymer of (meth)acrylic acid having an average molecular weight ranging from 100,000 to 850,000.

Preferably, the microgranules according to the present invention exhibit a granulometric distribution ranging between 50 and 700 µm.

The aforesaid copolymer may be anionic or cationic or neutral.

The anionic copolymer is preferably selected from the group consisting of:
a) a methacrylic acid-methyl methacrylate copolymer having an acid groups/esterified groups ratio of 1:1 and an average molecular weight of 135,000, such as the copolymer marketed under the trademark Eudragit® L100;
b) a methacrylic acid-ethyl methacrylate copolymer with an acid groups/esterified groups ratio of 1:1 and an average molecular weight of 250,000, such as the copolymer marketed under the trademark Eudragit® L100-55.

The cationic copolymer is preferably a butyl methacrylate-dimethylaminoethyl methacrylate-methyl methacrylate copolymer having for the three monomers the ratio of 1:2:1 respectively, and an average molecular weight of 150,000, such as the copolymer marketed under the trademark Eudragit® E.

The neutral copolymer is preferably an ethyl acrylate-methyl methacrylate copolymer having a 1:1 monomer ratio and an average molecular weight of 800,000.

In the microgranules of the invention the copolymer is preferably present in an amount ranging between 4 and 10% with respect to the amount of the active ingredient.

The plasticiser is preferably selected from the group consisting of glyceryl triacetate, triethyl citrate and polyethylene glycol 1500.

The microgranules of the invention may optionally include a lubricant, preferably talc or magnesium stearate.

The microgranules of the invention are tasteless and, as described in detail in the examples that follow, they have a dissolution profile substantially identical with that of pure ursodeoxycholic acid. Furthermore, the bioavailability of the formulations comprising the microgranules of the invention is substantially identical with that of the formulations comprising ursodeoxycholic acid as such.

It is a further subject of the present invention to provide a process for the preparation of the aforesaid microgranules comprising the following steps:
i) preparing an aqueous suspension comprising the copolymer, the plasticiser, and, optionally, the lubricant;
ii) feeding the ursodeoxycholic acid to a fluid bed granulator, and granulating by spraying the suspension as prepared in step i).

Preferably, the concentration of the suspension as prepared in step i) ranges between 10 and 20% w/v. Preferably, the said suspension comprises an amount of copolymer ranging from 4 to 10% with respect to the amount of ursodeoxycholic acid that is subjected to granulation in step ii), and a total amount of copolymer, plasticiser and lubricant ranging from 5 to 12% with respect to the amount of active ingredient in step ii). In fact the said per cent amounts of excipients were found to bring about microgranules that are tasteless and exhibit optimal characteristics for their subsequent utilisation, for example for the preparation of stable suspensions.

The granulation is preferably carried out under the following operating conditions:
inlet air temperature: 55° to 65°C,
inlet air rate: 1200 to 1400 rpm,
pump speed: 12 to 20 rpm,
spraying air pressure: 1.5 to 3 bar.

It is a further subject of the present invention to provide pharmaceutical formulations for the oral administration, comprising the aforesaid microgranules together with suitable excipients and/or diluents.

Amongst these formulations, particularly preferred are the powdered formulations for the extemporaneous preparation of suspensions and single-dose chewable formulations.

It is a further subject of the present invention to provide pharmaceutical formulations in form of suspensions, prepared by suspending the aforesaid powdered formulations in a suitable carrier, preferably in water.

If the microgranules of the invention contain an anionic or cationic copolymer, said excipients may comprise a pH adjuster. In the case of the aforesaid powdered formulations, the characteristics of the pH adjuster must be such as to bring the pH of the reconstituted suspension to a value which differs from that of solubilisation for the polymer. Instead, in the case of chewable formulations, the characteristics of the pH adjuster must be such as to hinder the copolymer solubilisation during its residence in the mouth. In particular, when the aforesaid copolymer is anionic, said pH adjuster is preferably a pharmaceutically acceptable acid, preferably selected from the group consisting of citric acid and tartaric acid.

To obtain a formulation with a pleasant taste, the aforesaid excipients may also comprise sweeteners and/or flavouring agents.

Preferred sweeteners are saccharose, mannitol, fructose, sorbitol and saccharin; amongst these, particularly preferred is mannitol, which provides good flowability to the product, in the case of powdered formulations.

According to a preferred embodiment of the invention, the excipients used in the preparation of the aforesaid powdered formulations for the extemporaneous preparation of suspensions, comprise at least a thickener capable of giving a sufficient physical stability to the reconstituted suspension. Said thickener is preferably selected from the group consisting of maize starch, microcrystalline cellulose, and sodium carboxymethylcellulose.

A particularly preferred powdered formulation comprises the microgranules of the invention in which the copolymer is anionic mixed with the following excipients: mannitol, monohydrated citric acid, sodium saccharinate, maize starch, microcrystalline cellulose and at least a flavouring agent.

A further preferred powdered formulation comprises microgranules in which the copolymer is cationic mixed with the following excipients: mannitol, sodium saccharinate, maize starch, microcrystalline cellulose and at least a flavouring agent.

A particularly preferred chewable formulation comprises the macrogranules of the invention in which the copolymer is anionic, sorbitol, citric acid, maltol and at least a flavouring agent.

The powdered formulations according to the present invention are prepared by dry mixing the components or according to other procedures known in the art. With a view to increasing the stability of the powder obtained upon the process completion, the moisture content is preferably ≤ 2%.

The powder to be reconstituted may be subdivided into single-dose packets or into multi-dose bottles, and, to obtain a suspension, is added to an adequate quantity of water just prior to use.

The chewable formulations of the invention are prepared by mixing the microgranules of the invention with suitable excipients followed by compression of the resulting powder.

The formulations of the invention preferably contain 150 or 300 mg ursodeoxycholic acid per unit dosage.

The following examples are conveyed by way of illustration, of the present invention.

### Example 1

A 30% suspension of Eudragit® L100-55 (8 kg) in water was added with triacetin (0.236 kg) and allowed to stir for 30 min. Separately, talc (0.236 kg) was suspended in water (7.5 l). This suspension was allowed to stir for 30 min, added to the suspension of Eudragit® and stirred vigorously for 5 min and then gently for additional 2 hrs. The resulting suspension was allowed to stand until foam disappearance. Ursodeoxycholic acid (30 kg) was fed to a fluid bed granulator. Granulation was performed by spraying the aforesaid suspension under the following operating conditions:
inlet air temperature: 60°C,
inlet air rate: 1,300 rpm,
pump speed: 15 rpm,
spraying air pressure: 2 bar.
32.872 kg of microgranules having a diameter comprised between 50 and 700 µm, were obtained.

### Example 2

The following powdered formulation, suitable for the extemporaneous preparation of suspensions, was prepared:

| | |
|---|---|
| microgranules prepared as per Example 1 | 32.872 kg |
| mannitol | 138.577 kg |
| monohydrated citric acid | 2.026 kg |
| sodium saccharinate | 0.385 kg |
| maize starch | 2.11 kg |
| Avicel® RC 581 | 21.1 kg |
| custard flavour | 2.93 kg |

### Procedure:

Mannitol, ursodeoxycholic acid microgranules, maize starch, citric acid, sodium saccharinate, Avicel® RC 581, and custard flavour were fed, in the aforesaid order, to a mixer and mixed for 30 min.

The granulate obtained was subdivided into 1 g packets, equivalent to 150 mg ursodeoxycholic acid, or into 2 g packets, equivalent to 300 mg ursodeoxycholic acid.

### Example 3

Chewable tablets having the following composition were prepared:

| | |
|---|---|
| microgranules prepared as per Example 1 | 32.872 kg |
| sorbitol | 112 kg |
| citric acid | 2.03 kg |
| maltol | 0.1 kg |
| caramel flavour | 2.36 kg |
| custard flavour | 0.64 kg |

### Procedure:

Ursodeoxycholic acid microgranules, citric acid, maltol, caramel flavour, custard flavour were fed, in the aforesaid order, to a Manes mixer and mixed for 30 min. The granulate was compressed to form chewable 1.5 g tablets, each containing 300 mg ursodeoxycholic acid.

### Example 4

Water (26.5 kg) was added with triacetin (0.432 kg), Eudragit® EPO (5 kg), talc (0.432 kg), sodium lauryl sulphate (0.304 kg), and Pharsil 21046 V.P. (30% simeticone) (0.505 kg). The suspension was stirred vigorously for 5 min, gently for additional 2 hrs, and allowed to stand until foam disappearance.

Ursodeoxycholic acid (30 kg) was fed to a fluid bed granulator. Granulation was performed by spraying the aforesaid suspension under the following operating conditions:
inlet air temperature: 60°C,
inlet air rate: 1,300 rpm,
pump speed: 15 rpm,
spraying air pressure: 2 bar.
35.9 kg of microgranules having a diameter ranging between 50 and 700 µm, was obtained.

### Example 5

The following formulation, suitable for the extemporaneous preparation of suspensions, was prepared:

| | |
|---|---|
| microgranules prepared as per Example 4 | 35.9 kg |
| mannitol | 136.357 kg |
| sodium saccharinate | 0.385 kg |
| maize starch | 2.11 kg |
| Avicel® RC 581 | 21.1 kg |
| custard flavour | 2.93 kg |

### Procedure:

Mannitol, ursodeoxycholic acid microgranules, maize starch, sodium saccharinate, Avicel® RC 581 and custard flavour were fed, in the aforesaid order, to a mixer and mixed for 30 min. The granulate obtained was subdivided into 1 g packets, equivalent to 150 mg ursodeoxycholic acid, or into 2 g packets, equivalent to 300 mg ursodeoxycholic acid.

### Example 6

Microgranules were prepared as per the procedure of Example 1, but with Eudragit® L100-55 replaced by Eudragit® NE 30 D.

### Example 7

### Evaluation of the dissolution rate of ursodeoxycholic acid microgranules depending on pH

The dissolution rate of the active ingredient from microgranules as per Example 1 was compared with the dissolution rate of the pure active ingredient, at different pH values.

Microgranules (2 g) as per Example 1 were fed to the vessel, thermostatted at 37°C±0.5°C, of a model AT6 Sotax® paddle dissolutor AT6, in 900 ml buffer solution, stirred at 100 rpm. The following buffer solutions were used for dissolution:
Buffer at pH 1.1:
   12 ml 37% HCI in 2 I water.
Buffer at pH 5.5:
   5.195 g KH₂PO₄ in 900 ml water, brought to pH 5.5 by addition of 10% H₃PO₄ and diluted with water to 1 l.
Buffer at pH 6:
   5.195 g KH₂PO₄ in 900 ml water, brought to pH 6 by addition of 1N NaOH and diluted with water to 1 l.
Buffer at pH 6.5:
   5.195 g KH₂PO₄ in 900 ml water, brought to pH 6.5 by addition of 1N NaOH and diluted with water to 1 l.
Buffer at pH 7.5:
   5.195 g KH₂PO₄ in 900 ml water, brought to pH 7.5 by addition of 1N NaOH and diluted with water to 1 l.
Buffer at pH 8.5:
   5.195 g KH₂PO₄ in 900 ml water, brought to pH 8.5 by addition of 1N NaOH and diluted with water to 1 l.

Samples of 20 ml were taken at 30 min intervals. The initial volume was made up each time with a buffer preheated to 37°C. The amount of active ingredient in the samples was evaluated by HPLC. In more detail, the samples were filtered and 10 ml filtrate was placed into a 20 ml flask, added with 3 ml internal standard prepared by diluting 100 mg cholic acid in 100 ml methanol. The volume was brought to 20 ml with methanol and, after stirring, 50 µl of the solution obtained was analysed by HPLC. The standard used was prepared as follows: 33.5 mg ursodeoxycholic acid was dissolved in 100 ml methanol. The resulting solution (10 ml) was added with 3 ml of the aforesaid internal standard and 7 ml methanol.

The so obtained results, expressed as per cent amount of active ingredient passed into solution after 2 hrs at different pH values, are showed in Figure 1.

### Example 8

### Bioavailability and bioequivalence study in healthy volunteers

The bioavailability of the formulation as per Example 2 in 150 mg and 300 mg packets was evaluated in healthy volunteers in comparison with the bioavailabiltiy of an ursodeoxycholic acid formulation in 150 mg and 300 mg capsules, available on commerce under the trademark URSILON®.

In particular, a randomised cross-over and open study was conducted on 24 healthy volunteers, 12 males and 12 females.

The volunteers, fasted for 8 hrs at least, received each of the four formulations at random, at least 7 days apart.

Prior to the administration of each ursodeoxycholic acid dose (time 0) and at 1, 2, 3, 4, 6, 8, and 24 hrs after drug administration, venous blood samples (approx. 7 ml) were taken by antecubital vein puncture in asepsis, under venous stasis condition.

The blood samples were transferred, immediately after taking, into heparinised test tubes, and centrifuged. The plasma was collected and stored at -20°C.

The ursodeoxycholic acid plasma levels in the various samples were determined by an immunoenzymatic method, formerly used and validated (Roda et al. 1994-1995, Simoni et al. 1995).

The determinations concerned:
- The areas under the concentration-time curves (AUC), estimated by the trapezoid method and extrapolation to the infinite, beyond the last experimental point under examination.
- The maximum concentration (Cmax).
- The maximum concentration time (Tmax) and the biological half-life (T_{1/2}).

### Results obtained

### 1) Bioequivalence

Table 1 shows the mean AUC and Cmax values (expressed as logarithms, LN) obtained in the 24 subjects, subdivided by treatment and dosage.

**Table 1:**

| AUC and Cmax of treatments (LN) | | | | | | | | |
|---|---|---|---|---|---|---|---|---|
| | **AUC** | | | | **Cmax** | | | |
| | Formulation Example 2 | | URSILON® | | Formulation Example 2 | | URSILON® | |
| Dose | Mean | ± SD | Mean | ± SD | Mean | ± SD | Mean | ± SD |
| 150 mg | 9.30 | 0.13 | 9.30 | 0.13 | 9.31 | 0.17 | 9.31 | 0.17 |
| 300 mg | 9.68 | 0.14 | 9.68 | 0.14 | 9.63 | 0.12 | 9.63 | 0.12 |

The analysis of variance showed a dose effect (p<0.01), which proves that the plasma concentrations closely depend on dosages and not on the type of formulation. No formulation-dose interaction was observed.

In the cross-over drawing, the examination of the variation sources, i.e. subjects (24), treatments (4), periods (4) and sequences (4), did not reveal any significant statistical difference due to the sequence and period effects, but confirmed the effect of the 4 treatments (p<0.01) on the basis of the different dosages.

The distribution of the treatment effect, shown in the cross-over drawing, was verified by paired t-test, between different dosages-formulations: no significant statistical difference was found, which proves that the effect is brought about by dosages and not by the type of formulation.

AUC and Cmax parameters were analysed by the Schuirman's test (two one-sided t-tests). The results obtained are shown in Table 2.

**Table 2 -**

| Schuirman's test results | | | | |
|---|---|---|---|---|
| Treatments | Parameters | Point estimate | Confidence interval (90%) | Test power |
| | AUC | 0.993 | 0.967-1.018 | >90% |
| Form. Ex.2 (150 mg) | | | | |
| URSILON® (300 mg) | | | | |
| | Cmax | 0.946 | 0.908-0.986 | >90% |
| | AUC | 1.054 | 1.000-1.108 | >90% |
| Form. Ex.2 (150 mg) | | | | |
| URSILON® (300 mg) | | | | |
| | Cmax | 1.005 | 0.964-1.047 | >90% |

The data obtained prove that the formulations under examination are bioequivalent since the confidence interval ranges from 0.8 to 1.25, with a >90% test power.

### 2) Tmax and T_{1/2}

Tmax evaluation, based on values expressed as hours, was performed by means of the non-parametric Friedman's test.

No significant statistical difference was observed; therefore, it may be inferred that the Tmax does not vary with the doses administered (Table 3).

**Table 3**

| Mean Tmax of treatments | | | | |
|---|---|---|---|---|
| | Formulation Example 2 | | URSILON® | |
| Dose | Mean | ± SD | Mean | ± SD |
| 150 mg | 3.04 | 0.75 | 2.88 | 0.68 |
| 300 mg | 2.67 | 0.56 | 2.63 | 0.49 |

Furthermore, the formulations in single doses were compared by the non-parametric Wilcoxon's method. No significant statistical difference was observed.

The data obtained prove that the Tmax values do not vary with the type of formulation administered.

The T_{1/2} values obtained with the various formulations were also analysed.

A substantial overlap was detected from the mean elimination values of the respective treatments (2.5 - 3 hrs). Therefore, a comparison by paired t-test was performed to verify the variation, if any, inside the single doses. Said comparison did not reveal any significant statistical difference. The data obtained prove that the different formulations do not modify the active ingredient disappearance rate.

## Claims

1. Microgranules for the oral administration of ursodeoxycholic acid, **characterised in that** they comprise, intimately mixed, ursodeoxycholic acid, a plasticiser and a pharmaceutically acceptable copolymer of (meth)acrylic acid having a molecular weight ranging between 100,000 and 850,000.

2. The microgranules according to claim 1, wherein the granulometric distribution of said microgranules ranges from 50 to 700 µm.

3. The microgranules according to claim 1, wherein said copolymer is an anionic or cationic or neutral copolymer.

4. The microgranules according to claim 3, wherein said anionic copolymer is selected from the group consisting of:
a) a methacrylic acid-methyl methacrylate copolymer with a ratio acid groups/esterified groups of 1:1 and an average molecular weight of 135,000;
b) a methacrylic acid-ethyl methacrylate copolymer with a ratio acid groups/esterified groups of 1:1 and an average molecular weight of 250,000.

5. The microgranules according to claim 3, wherein said cationic copolymer is a butyl methacrylate-dimethylaminoethyl methacrylate-methyl methacrylate copolymer with a 1:2:1 monomer ratio and an average molecular weight of 150,000.

6. The microgranules according to claim 3, wherein said neutral copolymer is an ethyl acrylate-methyl methacrylate copolymer with a 1:1 monomer ratio and an average molecular weight of 800,000.

7. The microgranules according to claim 1, wherein the amount of said copolymer ranges between 4 and 10% with respect to the amount of ursodeoxycholic acid.

8. The microgranules according to claim 1, wherein said plasticiser is selected from the group consisting of glyceryl triacetate, triethyl citrate and polyethylene glycol 1500.

9. The microgranules according to claim 1, wherein a lubricant is also comprised.

10. The microgranules according to claim 9, wherein said lubricant is selected from the group consisting of talc and magnesium stearate.

11. Procedure for the preparation of microgranules according to claim 1, comprising the following steps:
i) preparing an aqueous suspension including the copolymer, the plasticiser, and, optionally, the lubricant;
ii) feeding the ursodeoxycholic acid to a fluid bed granulator and granulating by spraying the suspension prepared in step i).

12. The procedure according to claim 11, wherein the granulation as per step ii) is carried out under the following operating conditions: inlet air temperature: 55° to 65°C; inlet air rate: 1200 to 1400 rpm; pump speed: 12 to 20 rpm; spraying air pressure: 1.5 to 3 bar.

13. The procedure according to claim 11, wherein the concentration of the suspension as prepared in step i) ranges between 10 and 20% w/v.

14. The procedure according to claim 11, wherein the amount of the copolymer in the suspension prepared in step i) is comprised between 4 and 10% with respect to the ursodeoxycholic acid as per step ii).

15. The procedure according to claim 11, wherein the total amount of copolymer, plasticiser and lubricant in the suspension as per step i) is comprised between 5 and 12% with respect to the ursodeoxycholic acid as per step ii).

16. Pharmaceutical formulation for oral administration comprising the microgranules according to claim 1 together with suitable excipients and/or diluents.

17. The formulation according to claim 16, comprising 150 or 300 mg of ursodeoxycholic acid per unit dosage.

18. The formulation according to claim 16, wherein said microgranules comprise an anionic or cationic copolymer and said excipients comprise a pH adjuster.

19. The formulation according to claim 18, wherein said copolymer is anionic and said pH adjuster is a pharmaceutically acceptable acid.

20. The formulation according to claim 19, wherein said pharmaceutically acceptable acid is citric acid or tartaric acid.

21. The formulation according to claim 16, in powder form for the extemporaneous preparation of suspensions.

22. The formulation according to claim 21, wherein said excipients comprise at least a thickener.

23. The formulation according to claim 22, wherein said thickener is preferably selected from the group consisting of maize starch, microcrystalline cellulose and sodium carboxymethylcellulose.

24. The formulation according to claim 21, wherein said microgranules comprise an anionic copolymer and said excipients comprise mannitol, monohydrated citric acid, sodium saccharinate, maize starch, microcrystalline cellulose, and a flavouring agent.

25. The formulation according to claim 21, wherein said microgranules comprise a cationic copolymer and said excipients comprise mannitol, sodium saccharinate, maize starch, microcrystalline cellulose, and a flavouring agent.

26. The formulation according to claim 16, obtained by suspending in water the formulation according to claim 21.

27. The formulation according to claim 16 in chewable tablet form.

28. The formulation according to claim 26, wherein said microgranules comprise an anionic copolymer and said excipients comprise sorbitol, citric acid, maltol, and at least a flavouring agent.

## Patentansprüche

1. Mikrogranulate für die orale Verabreichung von Ursodesoxycholinsäure, **dadurch gekennzeichnet, daß** sie in enger Vermischung Ursodesoxycholinsäure, einen Weichmacher und ein pharmazeutisch akzeptables Copolymer von (Meth)acrylsäure mit einem Molekulargewicht zwischen 100.000 und 850.000 umfassen.

2. Mikrogranulate gemäß Anspruch 1, worin die granulometrische Verteilung der Mikrogranulate im Bereich von 50 bis 700 µm liegt.

3. Mikrogranulate gemäß Anspruch 1, worin das Copolymer ein anionisches oder kationisches oder neutrales Copolymer ist.

4. Mikrogranulate gemäß Anspruch 3, worin das anionische Copolymer ausgewählt ist aus der Gruppe bestehend aus:
a) einem Methacrylsäure-Methylmethacrylat-Copolymer mit einem Verhältnis von Säuregruppen/veresterten Gruppen von 1:1 und einem durchschnittlichen Molekulargewicht von 135.000;
b) einem Methacrylsäure-Ethylmethacrylat-Copolymer mit einem Verhältnis von Säuregruppen/veresterten Gruppen von 1:1 und einem durchschnittlichen Molekulargewicht von 250.000.

5. Mikrogranulate gemäß Anspruch 3, worin das kationische Copolymer ein Butylmethacrylat-Dimethylaminoethylmethacrylat-Methylmethacrylat-Copolymer mit einem 1:2:1 Monomerverhältnis und einem durchschnittlichen Molekulargewicht von 150.000 ist.

6. Mikrogranulate gemäß Anspruch 3, worin das neutrale Copolymer ein Ethylacrylat-Methylmethacrylat-Copolymer mit einem 1:1 Monomerverhältnis und einem durchschnittlichen Molekulargewicht von 800.000 ist.

7. Mikrogranulate gemäß Anspruch 1, worin die Menge des Copolymers zwischen 4 und 10 % im Hinblick auf die Menge der Ursodesoxycholinsäure liegt.

8. Mikrogranulate gemäß Anspruch 1, worin der Weichmacher aus der Gruppe ausgewählt ist bestehend aus Glyceryltriacetat, Triethylcitrat und Polyethylenglycol 1500.

9. Mikrogranulate gemäß Anspruch 1, worin auch ein Gleitmittel umfaßt ist.

10. Mikrogranulate gemäß Anspruch 9, worin das Gleitmittel ausgewählt ist aus der Gruppe bestehend aus Talk und Magnesiumstearat.

11. Verfahren für die Herstellung von Mikrogranulaten gemäß Anspruch 1, umfassend die folgenden Schritte:
i) Herstellung einer wäßrigen Suspension beinhaltend das Copolymer, den Weichmacher und optional das Gleitmittel;
ii) Zufuhr der Ursodesoxycholinsäure zu einem Wirbelbettgranulator und Granulieren durch Versprühen der Suspension, die in Schritt i) hergestellt wurde.

12. Verfahren gemäß Anspruch 11, worin die Granulation gemäß Schritt ii) unter den folgenden Arbeitsbedingungen durchgeführt wird: Einlaßlufttemperatur: 55 bis 65°C; Einlaßluftrate: 1.200 bis 1.400 Upm; Pumpengeschwindigkeit: 12 bis 20 Upm; Sprühluftdruck: 1,5 bis 3 bar.

13. Verfahren gemäß Anspruch 11, worin die Konzentration der Suspension, die in Schritt i) hergestellt wurde, zwischen 10 und 20 % G/V liegt.

14. Verfahren gemäß Anspruch 11, worin die Menge des Copolymers in der in Schritt i) hergestellten Suspension zwischen 4 und 10 % im Hinblick auf die Ursodesoxycholinsäure gemäß Schritt ii) liegt.

15. Verfahren gemäß Anspruch 11, worin die Gesamtmenge des Copolymers, Weichmachers und Gleitmittels in der Suspension gemäß Schritt i) zwischen 5 und 12 % im Hinblick auf die Ursodesoxycholinsäure gemäß Schritt ii) liegt.

16. Pharmazeutische Formulierung für die orale Verabreichung, umfassend die Mikrogranulate gemäß Anspruch 1 zusammen mit geeigneten Exzipienten und/oder Verdünnungsmitteln.

17. Formulierung gemäß Anspruch 16, umfassend 150 bis 300 mg Ursodesoxycholinsäure pro Einheitsdosis.

18. Formulierung gemäß Anspruch 16, worin die Mikrogranulate ein anionisches oder kationisches Copolymer umfassen und wobei die Exzipienten ein pH-Einstellungsmittel umfassen.

19. Formulierung gemäß Anspruch 18, worin das Copolymer anionisch ist und worin das pH-Einstellungsmittel eine pharmazeutisch akzeptable Säure ist.

20. Formulierung gemäß Anspruch 19, worin die pharmazeutisch akzeptable Säure Zitronensäure oder Weinsäure ist.

21. Formulierung gemäß Anspruch 16 in Pulverform für die extemporäre Präparation von Suspensionen.

22. Formulierung gemäß Anspruch 21, worin die Exzipienten mindestens ein Verdickungsmittel umfassen.

23. Formulierung gemäß Anspruch 22, worin das Verdickungsmittel vorzugsweise aus der Gruppe gewählt wird bestehend aus Maisstärke, mikrokristalliner Cellulose und Natriumcarboxymethylcellulose.

24. Formulierung gemäß Anspruch 21, worin die Mikrogranulate ein anionisches Copolymer umfassen und wobei die Exzipienten Mannit, monohydratisierte Zitronensäure, Natriumsaccharinat, Maisstärke, mikrokristalline Cellulose und ein Geschmacksmittel umfassen.

25. Formulierung gemäß Anspruch 21, worin die Mikrogranulate ein kationisches Copolymer umfassen und wobei die Exzipienten Mannit, Natriumsaccharinat, Maisstärke, mikrokristalline Cellulose und ein Geschmacksmittel umfassen.

26. Formulierung gemäß Anspruch 16, erhalten durch Suspension der Formulierung gemäß Anspruch 21 in Wasser.

27. Formulierung gemäß Anspruch 16 in Kautablettenform.

28. Formulierung gemäß Anspruch 26, worin die Mikrogranulate ein anionisches Copolymer umfassen und wobei die Exzipienten Sorbit, Zitronensäure, Maltol und mindestens ein Geschmacksmittel umfassen.

## Revendications

1. Microgranules pour l'administration orale de l'acide ursodésoxycholique, **caractérisés en ce qu'**ils comprennent, en mélange intime, de l'acide ursodésoxycholique, un plastifiant, et un copolymère pharmaceutiquement acceptable d'acide (méth)acrylique ayant un poids moléculaire compris entre 100 000 et 850 000.

2. Microgranules selon la revendication 1, où la distribution granulométrique desdits microgranules va de 50 à 700 µm.

3. Microgranules selon la revendication 1, où ledit copolymère est un copolymère anionique ou cationique ou neutre.

4. Microgranules selon la revendication 3, où ledit copolymère anionique est sélectionné dans le groupe consistant en :
a) un copolymère d'acide méthacrylique-méthacrylate de méthyle avec un rapport des groupes acides/groupes estérifiés de 1:1 et un poids moléculaire moyen de 135000.
b) un copolymère d'acide méthacrylique-méthacrylate d'éthyle avec un rapport groupes acides/groupes estérifiés de 1:1 et un poids moléculaire moyen de 250 000.

5. Microgranules selon la revendication 3, où ledit copolymère cationique est un copolymère méthacrylate de butyle-méthacrylate de diméthyléminoéthyle-méthacrylate de méthyle avec un rapport des monomères de 1:2:1 et un poids moléculaire moyen de 150 000.

6. Microgranules selon la revendication 3, où ledit copolymère neutre est un copolymère d'acrylate d'éthyle-méthacrylate de méthyle avec un rapport des monomères de 1:1 et un poids moléculaire moyen de 800 000.

7. Microgranules selon la revendication 1, où la quantité dudit copolymère est comprise, entre 4 et 10% par rapport à la quantité de l'acide ursodesoxycholique.

8. Microgranules selon la revendication 1, où ledit plastifiant est sélectionné dans le groupe consistant en triacétate de glycéryle, citrate de triétyle et polyéthylène glycol 1500.

9. Microgranules selon la revendication 1, où un lubrifiant est également compris.

10. Microgranules selon la revendication 9, où ledit lubrifiant est sélectionné dans le groupe consistant en talc et stéarate de magnésium.

11. Processus pour la préparation de microgranules selon la revendication 1, comprenant les étapes suivantes :
i) préparation d'une suspension aqueuse comprenant le copolymère, le plastifiant, et facultativement, le lubrifiant ;
ii) fourniture de l'acide ursodésoxycholique à un granulateur à lit fluide et granulation par pulvérisation de la suspension préparée l'étape i).

12. Processus selon la revendication 11, où la granulation selon l'étape ii) est effectuée aux conditions de fonctionnement qui suivent : température d'entrée d'air : 55° à 65°C ; vitesse d'entrée de l'air 1200 à 1400 t/min; vitesse de la pompe : 12 à 20 t/min ; pression d'air de pulvérisation : 1,5 à 3 bars.

13. Processus selon la revendication 11, où la concentration de la suspension, telle que préparée à l'étape i), est comprise entre 10 et 20% p/v.

14. Processus selon la revendication 11, où la quantité du copolymère dans la suspension préparée à l'étape i) est comprise entre 4 et 10% par rapport à l'acide ursodésoxycholique selon l'étape ii).

15. Processus selon la revendication 11, où la quantité totale du copolymère, du plastifiant et du lubrifiant dans la suspension selon l'étape i) est comprise entre 5 et 12% par rapport à l'acide ursodésoxycholique selon l'étape ii).

16. Formulation pharmaceutique pour administration orale comprenant les microgranules selon la revendication 1, avec des excipients et/ou diluants appropriés.

17. Formulation selon la revendication 16, comprenant 150 ou 300 mg d'acide ursodésoxycholique par dosage unitaire.

18. Formulation selon la revendication 6, où lesdits microgranules comprennent un copolymère anionique ou cationique et lesdits excipients comprennent un moyen d'ajustement du pH.

19. Formulation selon la revendication 18, où ledit copolymère est anionique et ledit moyen d'ajustement du pH est un acide pharmaceutiquement acceptable.

20. Formulation selon la revendication 19, où ledit acide pharmaceutiquement acceptable est l'acide citrique ou l'acide tartrique.

21. Formulation selon la revendication 16, en forme de poudre pour la préparation extemporanée de suspensions.

22. Formulation selon la revendication 21, où lesdits excipients comprennent au moins un épaississant.

23. Formulation selon la revendication 22, où ledit épaississant est de préférence sélectionné dans le groupe consistant en amidon de maïs, cellulose microcristalline et sodium carboxyméthylcellulose.

24. Formulation selon la revendication 21, où lesdits microgranules comprennent un copolymère anionique et lesdits excipients comprennent mannitol, acide citrique monohydraté, saccharinate de sodium, amidon de maïs, cellulose microcristalline et un agent aromatisant.

25. Formulation selon la revendication 21, où lesdits microgranules comprennent un copolymère cationique et lesdits excipients comprennent mannitol, saccharinate de sodium, amidon de maïs, cellulose microcristalline et un agent aromatisant.

26. Formulation selon la revendication 16, obtenue par mise en suspension dans l'eau de la formulation selon la revendication 21.

27. Formulation selon la revendication 16 sous forme de comprimés mâchables.

28. Formulation selon la revendication 26, où lesdits microgranules comprennent un copolymère anionique et lesdits excipients comprennent sorbitol, acide citrique, maltol et au moins un agent aromatisant.
